# EUROPÄISCHE PATENTSCHRIFT

(11) **EP 1 203 078 B1**
(45) Veröffentlichungstag und Bekanntmachung des Hinweises auf die Patenterteilung: **04.05.2005**
(21) Anmeldenummer: 00954320.8
(22) Anmeldetag: 08.07.2000
(51) Int. Cl.: C12N 15/00

(54) **IMPFSTOFF GEGEN INFEKTIONEN MIT LENTIVIREN, WIE DEM FELINEN IMMUNSCHWÄCHE-VIRUS DER KATZE**
VACCINE AGAINST LENTIVIRAL INFECTIONS, SUCH AS THE FELINE IMMUNE DEFICIENCY VIRUS OF THE CAT
VACCIN CONTRE LES INFECTIONS AU LENTIVIRUS COMME LE VIRUS DE L'IMMUNODEFICIENCE FELINE DU CHAT

(30) Priorität: 08.07.1999 CH 125899
(43) Veröffentlichungstag der Anmeldung: 08.05.2002
(73) Patentinhaber: Mologen Forschungs-, Entwicklungs- und Vertriebs GmbH, 14195 Berlin (DE); Universität Zürich, 8001 Zürich (CH)
(72) Erfinder: LEUTENEGGER, Christian, Ch-8610 Uster (CH); SCHROFF, Matthias, D-14057 Berlin (DE); WITTIG, Burghardt, D-14197 Berlin (DE); LUTZ, Hans, Ch-8455 Rüdlingen (CH)
(74) Vertreter: Vialle-Presles, Marie José
(86) Internationale Anmeldenummer: PCT/DE2000/002262
(87) Internationale Veröffentlichungsnummer: WO 2001/004280

(56) Entgegenhaltungen:
- WO-A-94/06471
- WO-A-95/30019
- FR-A- 2 751 223
- US-A- 5 571 515
- HOSIE M J ET AL: "DNA vaccination affords significant protection against feline immunodeficiency virus infection without inducing detectable antiviral antibodies." JOURNAL OF VIROLOGY, Bd. 72, Nr. 9, September 1998 (1998-09), Seiten 7310-7319, XP000944624
- FEHR D ET AL: "Nucleotide and predicted peptide sequence of feline interleukin-12 (IL-12)." DNA SEQUENCE, (1997) 8 (1-2) 77-82. , XP000944982
- CUISINIER A M ET AL: "Attempt to modify the immune response developed against FIV gp120 protein by preliminary FIV DNA injection" VACCINE,GB,BUTTERWORTH SCIENTIFIC. GUILDFORD, Bd. 17, Nr. 5, Februar 1999 (1999-02), Seiten 415-425, XP004153823 ISSN: 0264-410X
- LEUTENEGGER C M ET AL: "Molecular cloning and expression of feline interleukin-16." DNA SEQUENCE, (1998 MAR) 9 (1) 59-63. , XP000944981

### SEQUENZPROTOKOLL

<110> Mologen Forschungs-, Entwicklungs- und Vertriebs G Universität Zürich
<120> Impfstoff gegen Infektionen mit Lentiviren, wie dem felinen Immunschwäche-Virus der Katze
<130> XI 1057/00
<140>
   <141>
<150> CH 1999 1258/99
   <151> 1999-07-08
<160> 10
<170> PatentIn Ver. 2.1
<210> 1
   <211> 5871
   <212> DNA
   <213> Künstliche Sequenz
<220>
   <223> Beschreibung der künstlichen Sequenz: FIV gp140 (pMOLFIVgp140)
<400> 1
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttgcga attctggatc cgctagctta accgtattac cgccatgcat tagttattaa 2460
   tagtaatcaa ttacggggtc attagttcat agcccatata tggagttccg cgttacataa 2520
   cttacggtaa atggcccgcc tggctgaccg cccaacgacc cccgcccatt gacgtcaata 2580
   atgacgtatg ttcccatagt aacgccaata gggactttcc attgacgtca atgggtggag 2640
   tatttacggt aaactgccca cttggcagta catcaagtgt atcatatgcc aagtacgccc 2700
   cctattgacg tcaatgacgg taaatggccc gcctggcatt atgcccagta catgacctta 2760
   tgggactttc ctacttggca gtacatctac gtattagtca tcgctattac catggtgatg 2820
   cggttttggc agtacatcaa tgggcgtgga tagcggtttg actcacgggg atttccaagt 2880
   ctccacccca ttgacgtcaa tgggagtttg ttttggcacc aaaatcaacg ggactttcca 2940
   aaatgtcgta acaactccgc cccattgacg caaatgggcg gtaggcgtgt acggtgggag 3000
   gtctatataa gcagagctgg tttagtgaac cgtcagatgg taccatggca gaaggatttg 3060
   cagccaatag acaatggata gggccagaag aagctgaagg gttgttagat tttgatatag 3120
   caacacaaat gaatgaagaa gggccactaa atccaggaat aaacccattt agggtgcctg 3180
   gaatagcaga aatagaaaag cgagactatt gcaaaatatt acaacccaaa ttacaagatc 3240
   taaagaatga aattcaagag gtaaaactgg aagaaggaaa tgcaggtaag tttagaagag 3300
   caagattttt aagatattct gatgaaaata tattatccct gattcatttg ttcatagggt 3360
   attgtacata tttatgcaga aaaaatgagt taggatcttt acgacatgac atagatatag 3420
   acgaacatca agaagagtat tatactagta tagagaaagg tacaactgcc aatataaaat 3480
   atggtagacg atgtctcata ggaacagcgg ctttgtacct gcttttcata ggaataataa 3540
   tatatacaca aacaaccaag gctcaggtag tatggagact tccaccatta gtagtccccg 3600
   tgaaagaatc agagataatt ttttgggatt gttgggcacc agaggaaccc gcctgtcagg 3660
   actttcttgg ggcaatgata catctaaaag ctagtacaaa tataagtata caagagggac 3720
   ctaccctggg gaattgggct agagaaatat ggggaacatt attcaaaaag gctaccagac 3780
   aatgtagaag aggtagagta tggagaagat ggaatgagac tataacagga ccatcaggat 3840
   gtgctaataa cacatgttat aatatctcag taatagtacc tgattatcaa tgttatttag 3900
   acagagtaga tacttggtta caagggaaag taaatatatc attatgtcta acaggaggaa 3960
   aaatgttgta taataaatat acaaaacaat tgagctattg tacagatcca ttacaaatcc 4020
   cactgatcaa ttatacgttt ggacctaatc aaacatgtaa gtggaacact tcacagattc 4080
   aagactctga gataccaaaa tgtggatggt ggaatcaagc agcctattat aacagttgta 4140
   gatgggaaag cactgatgta aagtttcatt gtcaaagaac acagagtctg cctggaacat 4200
   ggcttagaac aatctcatca tggaggccaa agaatagatg ggaatggagg ccagattttg 4260
   aaagtgaaaa agtgaaagta tctctacagt gtaatagcac aagcaaccta acctttgcaa 4320
   tgagaagttc aggagattat ggagaggtaa cgggagcatg gatagaattt ggatgtcata 4380
   ggaaaaaatc aaaacttcat tctgaagcaa ggtttagaat cagatgtaga tgggataaag 4440
   gggataatac ctcactcatt gatacatgtg gaaaaactca aaatgtttta ggtgcaaatc 4500
   ctgtagattg caccatgtat gcaaatagaa tgtataattg ttccttacaa aatgggttta 4560
   ctatgaagat agatgacctt gttatgcatt tcaatatgac gaaagctgta gaaatgtata 4620
   acattgctgg aaattggtct tgtacatctg acttgccacc aacatggggg tatatgaatt 4680
   gtaattgtac aaatagtagt agtacaacta gtagttctgg taataaaatg gcatgtcctg 4740
   gagataaagg tatcttaaga aattggtata acccagtagc aggattaaga caatccctag 4800
   aaaagtatca agtagtaaaa caaccagatt acttagtggt gccaggggaa gtcatggaat 4860
   ataaacctag aaggaaaaga gcagctattc atgttatgtt agctcttgca acagtattat 4920
   ctatggccgg ggcagggacg ggggctactg ctatagggat ggtaacgcaa tatcaccaag 4980
   ttctggcaac tcatcaagaa gctatagaaa aggtgactga agccttaaag ataaacaact 5040
   taagattagt tacattagag catcaagtac tagtaatagg attaaaagta gaagctatgg 5100
   aaaaattttt atatacagct ttcgctatgc aagaattagg atgtaatcaa aatcaattct 5160
   tctgtaaagt ccctcctgtg ttgtgggaaa gatataatat gactataaat caaacaatat 5220
   ggaatcatgg aaatataact ttgggggaat ggtataacca aacaaaagat ttacaacaaa 5280
   agttctatga aataataatg gacatagaac aaaataatgt acaaggaaaa aaagggttac 5340
   aacaattaca aaaatgggaa gattgggtag gatggatagg aaatattcca aaatatttat 5400
   aagagctcat aatcagccat accacatttg tagaggtttt acttgcttta aaaaacctcc 5460
   cacacctccc cctgaacctg aaacataaaa tgaatgcaat tcttgttgtt aacttgttta 5520
   ttgcagctta taatggttac aaataaagca atagcatcac aaatttcaca aataaagcat 5580
   ttttttcact gcattctagt tgtggtttgt ccaaactcat caatgtatct taacgcgaat 5640
   tcgtaatcat ggtcatagct gtttcctgtg tgaaattgtt atccgctcac aattccacac 5700
   aacatacgag ccggaagcat aaagtgtaaa gcctggggtg cctaatgagt gagctaactc 5760
   acattaattg cgttgcgctc actgcccgct ttccagtcgg gaaacctgtc gtgccagctg 5820
   cattaatgaa tcggccaacg cgcggggaga ggcggtttgc gtattgggcg c          5871
<210> 2
   <211> 4522
   <212> DNA
   <213> Künstliche Sequenz
<220>
   <223> Beschreibung der künstlichen Sequenz: IL-12p40
<400> 2
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttggtc tccccctgga tccgctagct taaccgtatt accgccatgc attagttatt 2460
   aatagtaatc aattacgggg tcattagttc atagcccata tatggagttc cgcgttacat 2520
   aacttacggt aaatggcccg cctggctgac cgcccaacga cccccgccca ttgacgtcaa 2580
   taatgacgta tgttcccata gtaacgccaa tagggacttt ccattgacgt caatgggtgg 2640
   agtatttacg gtaaactgcc cacttggcag tacatcaagt gtatcatatg ccaagtacgc 2700
   cccctattga cgtcaatgac ggtaaatggc ccgcctggca ttatgcccag tacatgacct 2760
   tatgggactt tcctacttgg cagtacatct acgtattagt catcgctatt accatggtga 2820
   tgcggttttg gcagtacatc aatgggcgtg gatagcggtt tgactcacgg ggatttccaa 2880
   gtctccaccc cattgacgtc aatgggagtt tgttttggca ccaaaatcaa cgggactttc 2940
   caaaatgtcg taacaactcc gccccattga cgcaaatggg cggtaggcgt gtacggtggg 3000
   aggtctatat aagcagagct ggtttagtga accgtcagat ggtaccatgc atcctcagca 3060
   gttggtcatc gcctggtttt ccctggtttt gctggcacct cccctcatgg ccatatggga 3120
   actggagaaa aacgtttatg ttgtagagtt ggactggcac cctgatgccc ccggagaaat 3180
   ggtggtcctt acctgcaata ctcctgaaga agatgacatc acctggacct ctgaccagag 3240
   cagtgaagtc ctaggctctg gtaaaactct gaccatccaa gtcaaagaat ttgcagatgc 3300
   tggccagtat acctgtcata aaggaggcga ggttctgagc cattcgttcc tcctgataca 3360
   caaaaaggaa gatggaattt ggtccactga tatcttaagg gaacagaaag aatccaaaaa 3420
   taagatcttt ctaaaatgtg aggcaaagaa ttattctgga cgtttcacct gctggtggct 3480
   gacggcaatc agtaccgatt tgaaattcac tgtcaaaagc agcagaggct cctctgaccc 3540
   ccaaggggtg acttgtggag cagcgacact ctcagcagag aaggtcagag tggacaacag 3600
   ggattataag aagtacacag tggagtgtca ggagggcagt gcctgcccgg ctgccgagga 3660
   gagcctaccc attgaagtcg tggtggacgc tattcacaag ctcaagtacg aaaactacac 3720
   cagcagcttc ttcatcaggg acatcatcaa accggaccca cccaagaacc tgcaactgaa 3780
   gccattaaaa aattctcggc atgtggaagt gagctgggaa taccctgaca cctggagcac 3840
   cccacattcc tacttctcct taacatttgg cgtacaggtc cagggcaaga acaacagaga 3900
   aaagaaagac agactctccg tggacaagac ctcagccaag gtcgtgtgcc acaaggatgc 3960
   caagatccgc gtgcaagcca gggaccgcta ctatagctca tcctggagca actgggcatc 4020
   cgtgtcctgc agttaggagc tcataatcag ccataccaca tttgtagagg ttttacttgc 4080
   tttaaaaaac ctcccacacc tccccctgaa cctgaaacat aaaatgaatg caattcttgt 4140
   tgttaacttg tttattgcag cttataatgg ttacaaataa agcaatagca tcacaaattt 4200
   cacaaataaa gcattttttt cactgcattc tagttgtggt ttgtccaaac tcatcaatgt 4260
   atcttaacgc gaattcaggg ggagacccaa ttcgtaatca tggtcatagc tgtttcctgt 4320
   gtgaaattgt tatccgctca caattccaca caacatacga gccggaagca taaagtgtaa 4380
   agcctggggt gcctaatgag tgagctaact cacattaatt gcgttgcgct cactgcccgc 4440
   tttccagtcg ggaaacctgt cgtgccagct gcattaatga atcggccaac gcgcggggag 4500
   aggcggtttg cgtattgggc gc                                          4522
<210> 3
   <211> 4201
   <212> DNA
   <213> Künstliche Sequenz
<220>
   <223> Beschreibung der künstlichen Sequenz: IL-12p35 (pMOLfIL-12p35 (e-,_e31-)clone61)
<400> 3
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttggtc tccccctgga tccgctagct taaccgtatt accgccatgc attagttatt 2460
   aatagtaatc aattacgggg tcattagttc atagcccata tatggagttc cgcgttacat 2520
   aacttacggt aaatggcccg cctggctgac cgcccaacga cccccgccca ttgacgtcaa 2580
   taatgacgta tgttcccata gtaacgccaa tagggacttt ccattgacgt caatgggtgg 2640
   agtatttacg gtaaactgcc cacttggcag tacatcaagt gtatcatatg ccaagtacgc 2700
   cccctattga cgtcaatgac ggtaaatggc ccgcctggca ttatgcccag tacatgacct 2760
   tatgggactt tcctacttgg cagtacatct acgtattagt catcgctatt accatggtga 2820
   tgcggttttg gcagtacatc aatgggcgtg gatagcggtt tgactcacgg ggatttccaa 2880
   gtctccaccc cattgacgtc aatgggagtt tgttttggca ccaaaatcaa cgggactttc 2940
   caaaatgtcg taacaactcc gccccattga cgcaaatggg cggtaggcgt gtacggtggg 3000
   aggtctatat aagcagagct ggtttagtga accgtcagat ggtaccatgt gcccgccgcg 3060
   tggcctcctc cttgtaacca tcctggtcct gttaaaccac ctggaccacc tcagtttggc 3120
   caggaacctc cccacaccca caccaagccc aggaatgttc cagtgcctca accactccca 3180
   aaccctgctg cgagccatca gcaacacgct tcagaaggcc agacaaactc tagaatttta 3240
   cccctgcact tccgaagaga ttgatcatga agatatcaca aaagataaaa ccagcacagt 3300
   ggaggcctgc ttaccactgg aattagccat gaatgagagt tgcctggctt ccagagagat 3360
   ctctctgata actaatggga gttgcctggt gtccagaaag acctctttta tgacgaccct 3420
   gtgccttagc agtatctatg aggacttgaa gatgtaccag gtggagttca aggccatgaa 3480
   tgcaaagctg ttaatggatc ctaaaaggca gatctttctg gatcaaaaca tgctgacagc 3540
   tattgatgag ctgatgcagg ccctgaattt caacagtgtg actgtgccac agaactcctc 3600
   ccttgaagaa ccggattttt ataaaactaa aatcaagctc tgcatacttc ttcatgcttt 3660
   cagaatccgt gcagtgacca tcaatagaat gatgagctat ctgaatgctt cctaggagct 3720
   cataatcagc cataccacat ttgtagaggt tttacttgct ttaaaaaacc tcccacacct 3780
   ccccctgaac ctgaaacata aaatgaatgc aattgttgtt gttaacttgt ttattgcagc 3840
   ttataatggt tacaaataaa gcaatagcat cacaaatttc acaaataaag catttttttc 3900
   actgcattct agttgtggtt tgtccaaact catcaatgta tcttaacgcg aattcagggg 3960
   gagacccaat tcgtaatcat ggtcatagct gtttcctgtg tgaaattgtt atccgctcac 4020
   aattccacac aacatacgag ccggaagcat aaagtgtaaa gcctggggtg cctaatgagt 4080
   gagctaactc acattaattg cgttgcgctc actgcccgct ttccagtcgg gaaacctgtc 4140
   gtgccagctg cattaatgaa tcggccaacg cgcggggaga ggcggtttgc gtattgggcg 4200
   c                                                                 4201
<210> 4
   <211> 3903
   <212> DNA
   <213> Künstliche Sequenz
<220>
   <223> Beschreibung der künstlichen Sequenz: IL-16
<400> 4
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttgcga attctggatc cgctagctta accgtattac cgccatgcat tagttattaa 2460
   tagtaatcaa ttacggggtc attagttcat agcccatata tggagttccg cgttacataa 2520
   cttacggtaa atggcccgcc tggctgaccg cccaacgacc cccgcccatt gacgtcaata 2580
   atgacgtatg ttcccatagt aacgccaata gggactttcc attgacgtca atgggtggag 2640
   tatttacggt aaactgccca cttggcagta catcaagtgt atcatatgcc aagtacgccc 2700
   cctattgacg tcaatgacgg taaatggccc gcctggcatt atgcccagta catgacctta 2760
   tgggactttc ctacttggca gtacatctac gtattagtca tcgctattac catggtgatg 2820
   cggttttggc agtacatcaa tgggcgtgga tagcggtttg actcacgggg atttccaagt 2880
   ctccacccca ttgacgtcaa tgggagtttg ttttggcacc aaaatcaacg ggactttcca 2940
   aaatgtcgta acaactccgc cccattgacg caaatgggcg gtaggcgtgt acggtgggag 3000
   gtctatataa gcagagctgg tttagtgaac cgtcagatgg taccatgccc gacctcaact 3060
   cctccactga ttctacaaac tcggcttctg tggccagcga cgtttctggg gattccacgg 3120
   aggccacggt gcacacggtg acgctggaga agacgtccgc ggggctgggc ttcagcctgg 3180
   aaggcggcaa gggctccctg ctcggggaca agcctctcac cgtgaacagg attttcaaag 3240
   gggcagcctc ggaacagagc gagacgatcc agccgggaga tgaaatctta cacttggccg 3300
   gcactgccgt gcaggggctc acgcggtttg aagcctggaa cgttatcaag acgttgcctg 3360
   acggccccgt cacgatcgtc atcaggagga gaagcgtcca gtcctcggga accacagctg 3420
   ctggagactc ctaggagctc ataatcagcc ataccacatt tgtagaggtt ttacttgctt 3480
   taaaaaacct cccacacctc cccctgaacc tgaaacataa aatgaatgca attcttgttg 3540
   ttaacttgtt tattgcagct tataatggtt acaaataaag caatagcatc acaaatttca 3600
   caaataaagc atttttttca ctgcattcta gttgtggttt gtccaaactc atcaatgtat 3660
   cttaacgcga attcgtaatc atggtcatag ctgtttcctg tgtgaaattg ttatccgctc 3720
   acaattccac acaacatacg agccggaagc ataaagtgta aagcctgggg tgcctaatga 3780
   gtgagctaac tcacattaat tgcgttgcgc tcactgcccg ctttccagtc gggaaacctg 3840
   tcgtgccagc tgcattaatg aatcggccaa cgcgcgggga gaggcggttt gcgtattggg 3900
   cgc                                                               3903
<210> 5
   <211> 33
   <212> DNA
   <213> Künstliche Sequenz
<220>
   <223> Beschreibung der künstlichen Sequenz: CpG
<400> 5
   gttcttcggg gcgttctttt ttaagaacgc ccc 33
<210> 6
   <211> 35
   <212> DNA
   <213> Künstliche Sequenz
<220>
   <223> Beschreibung der künstlichen Sequenz: CpG
<400> 6
   gaagaacgtt ttccaatgat ttttcattgg aaaac 35
<210> 7
   <211> 2358
   <212> DNA
   <213> Künstliche Sequenz
<220>
   <223> Beschreibung der künstlichen Sequenz: Sequence coding for SEQ ID NO 1, FIV env gene
<400> 7
   atggcagaag gatttgcagc caatagacaa tggatagggc cagaagaagc tgaagggttg 60
   ttagattttg atatagcaac acaaatgaat gaagaagggc cactaaatcc aggaataaac 120
   ccatttaggg tgcctggaat agcagaaata gaaaagcgag actattgcaa aatattacaa 180
   cccaaattac aagatctaaa gaatgaaatt caagaggtaa aactggaaga aggaaatgca 240
   ggtaagttta gaagagcaag atttttaaga tattctgatg aaaatatatt atccctgatt 300
   catttgttca tagggtattg tacatattta tgcagaaaaa atgagttagg atctttacga 360
   catgacatag atatagacga acatcaagaa gagtattata ctagtataga gaaaggtaca 420
   actgccaata taaaatatgg tagacgatgt ctcataggaa cagcggcttt gtacctgctt 480
   ttcataggaa taataatata tacacaaaca accaaggctc aggtagtatg gagacttcca 540
   ccattagtag tccccgtgaa agaatcagag ataatttttt gggattgttg ggcaccagag 600
   gaacccgcct gtcaggactt tcttggggca atgatacatc taaaagctag tacaaatata 660
   agtatacaag agggacctac cctggggaat tgggctagag aaatatgggg aacattattc 720
   aaaaaggcta ccagacaatg tagaagaggt agagtatgga gaagatggaa tgagactata 780
   acaggaccat caggatgtgc taataacaca tgttataata tctcagtaat agtacctgat 840
   tatcaatgtt atttagacag agtagatact tggttacaag ggaaagtaaa tatatcatta 900
   tgtctaacag gaggaaaaat gttgtataat aaatatacaa aacaattgag ctattgtaca 960
   gatccattac aaatcccact gatcaattat acgtttggac ctaatcaaac atgtaagtgg 1020
   aacacttcac agattcaaga ctctgagata ccaaaatgtg gatggtggaa tcaagcagcc 1080
   tattataaca gttgtagatg ggaaagcact gatgtaaagt ttcattgtca aagaacacag 1140
   agtctgcctg gaacatggct tagaacaatc tcatcatgga ggccaaagaa tagatgggaa 1200
   tggaggccag attttgaaag tgaaaaagtg aaagtatctc tacagtgtaa tagcacaagc 1260
   aacctaacct ttgcaatgag aagttcagga gattatggag aggtaacggg agcatggata 1320
   gaatttggat gtcataggaa aaaatcaaaa cttcattctg aagcaaggtt tagaatcaga 1380
   tgtagatggg ataaagggga taatacctca ctcattgata catgtggaaa aactcaaaat 1440
   gttttaggtg caaatcctgt agattgcacc atgtatgcaa atagaatgta taattgttcc 1500
   ttacaaaatg ggtttactat gaagatagat gaccttgtta tgcatttcaa tatgacgaaa 1560
   gctgtagaaa tgtataacat tgctggaaat tggtcttgta catctgactt gccaccaaca 1620
   tgggggtata tgaattgtaa ttgtacaaat agtagtagta caactagtag ttctggtaat 1680
   aaaatggcat gtcctggaga taaaggtatc ttaagaaatt ggtataaccc agtagcagga 1740
   ttaagacaat ccctagaaaa gtatcaagta gtaaaacaac cagattactt agtggtgcca 1800
   ggggaagtca tggaatataa acctagaagg aaaagagcag ctattcatgt tatgttagct 1860
   cttgcaacag tattatctat ggccggggca gggacggggg ctactgctat agggatggta 1920
   acgcaatatc accaagttct ggcaactcat caagaagcta tagaaaaggt gactgaagcc 1980
   ttaaagataa acaacttaag attagttaca ttagagcatc aagtactagt aataggatta 2040
   aaagtagaag ctatggaaaa atttttatat acagctttcg ctatgcaaga attaggatgt 2100
   aatcaaaatc aattcttctg taaagtccct cctgtgttgt gggaaagata taatatgact 2160
   ataaatcaaa caatatggaa tcatggaaat ataactttgg gggaatggta taaccaaaca 2220
   aaagatttac aacaaaagtt ctatgaaata ataatggaca tagaacaaaa taatgtacaa 2280
   ggaaaaaaag ggttacaaca attacaaaaa tgggaagatt gggtaggatg gataggaaat 2340
   attccaaaat atttataa                                               2358
<210> 8
   <211> 990
   <212> DNA
   <213> Künstliche Sequenz
<220>
   <223> Beschreibung der künstlichen Sequenz: Sequence coding for SEQ ID NO 2, IL-12 p40
<400> 8
   atgcatcctc agcagttggt catcgcctgg ttttccctgg ttttgctggc acctcccctc 60
   atggccatat gggaactgga gaaaaacgtt tatgttgtag agttggactg gcaccctgat 120
   gcccccggag aaatggtggt ccttacctgc aatactcctg aagaagatga catcacctgg 180
   acctctgacc agagcagtga agtcctaggc tctggtaaaa ctctgaccat ccaagtcaaa 240
   gaatttgcag atgctggcca gtatacctgt cataaaggag gcgaggttct gagccattcg 300
   ttcctcctga tacacaaaaa ggaagatgga atttggtcca ctgatatctt aagggaacag 360
   aaagaatcca aaaataagat ctttctaaaa tgtgaggcaa agaattattc tggacgtttc 420
   acctgctggt ggctgacggc aatcagtacc gatttgaaat tcactgtcaa aagcagcaga 480
   ggctcctctg acccccaagg ggtgacttgt ggagcagcga cactctcagc agagaaggtc 540
   agagtggaca acagggatta taagaagtac acagtggagt gtcaggaggg cagtgcctgc 600
   ccggctgccg aggagagcct acccattgaa gtcgtggtgg acgctattca caagctcaag 660
   tacgaaaact acaccagcag cttcttcatc agggacatca tcaaaccgga cccacccaag 720
   aacctgcaac tgaagccatt aaaaaattct cggcatgtgg aagtgagctg ggaataccct 780
   gacacctgga gcaccccaca ttcctacttc tccttaacat ttggcgtaca ggtccagggc 840
   aagaacaaca gagaaaagaa agacagactc tccgtggaca agacctcagc caaggtcgtg 900
   tgccacaagg atgccaagat ccgcgtgcaa gccagggacc gctactatag ctcatcctgg 960
   agcaactggg catccgtgtc ctgcagttag                                  990
<210> 9
   <211> 669
   <212> DNA
   <213> Künstliche Sequenz
<220>
   <223> Beschreibung der künstlichen Sequenz: Sequence coding for SEQ ID NO 3, IL-12 p35
<400> 9
   atgtgcccgc cgcgtggcct cctccttgta accatcctgg tcctgttaaa ccacctggac 60
   cacctcagtt tggccaggaa cctccccaca cccacaccaa gcccaggaat gttccagtgc 120
   ctcaaccact cccaaaccct gctgcgagcc atcagcaaca cgcttcagaa ggccagacaa 180
   actctagaat tttacccctg cacttccgaa gagattgatc atgaagatat cacaaaagat 240
   aaaaccagca cagtggaggc ctgcttacca ctggaattag ccatgaatga gagttgcctg 300
   gcttccagag agatctctct gataactaat gggagttgcc tggtgtccag aaagacctct 360
   tttatgacga ccctgtgcct tagcagtatc tatgaggact tgaagatgta ccaggtggag 420
   ttcaaggcca tgaatgcaaa gctgttaatg gatcctaaaa ggcagatctt tctggatcaa 480
   aacatgctga cagctattga tgagctgatg caggccctga atttcaacag tgtgactgtg 540
   ccacagaact cctcccttga agaaccggat ttttataaaa ctaaaatcaa gctctgcata 600
   cttcttcatg ctttcagaat ccgtgcagtg accatcaata gaatgatgag ctatctgaat 660
   gcttcctag                                                          669
<210> 10
   <211> 390
   <212> DNA
   <213> Künstliche Sequenz
<220>
   <223> Beschreibung der künstlichen Sequenz: Sequence coding for SEQ ID NO 4, IL-16
<400> 10
   atgcccgacc tcaactcctc cactgattct acaaactcgg cttctgtggc cagcgacgtt 60
   tctggggatt ccacggaggc cacggtgcac acggtgacgc tggagaagac gtccgcgggg 120
   ctgggcttca gcctggaagg cggcaagggc tccctgctcg gggacaagcc tctcaccgtg 180
   aacaggattt tcaaaggggc agcctcggaa cagagcgaga cgatccagcc gggagatgaa 240
   atcttacact tggccggcac tgccgtgcag gggctcacgc ggtttgaagc ctggaacgtt 300
   atcaagacgt tgcctgacgg ccccgtcacg atcgtcatca ggaggagaag cgtccagtcc 360
   tcgggaacca cagctgctgg agactcctag                                  390

## Beschreibung

### Technisches Gebiet

Die vorliegende Erfindung bezieht sich auf einen Impfstoff, mit dem Katzen gegen die Infektion mit dem felinen Immunschwäche-Virus geschützt werden können, insbesondere auf einen Impfstoff auf DNA-Basis.

### Stand der Technik

Im Jahre 1987 wurde erstmals bei Hauskatzen ein Virus isoliert, welches bei infizierten Tieren zu einer Schwächung des Immunsystems führt (Pedersen et al., 1987). Bei diesem Virus handelt es sich um das feline Immunschwäche-Virus (FIV), welches als Lentivirus zu den Retroviren gehört. Das FIV ist nahe verwandt mit dem humanen Immunschwäche-Virus (HIV), mit dem simian Immunschwäche-Virus der Affen (SIV), mit den Lentiviren des Pferdes (equines infektiöses Anämievirus. EIAV) und der kleinen Wiederkäuer (Maedi-Visna-Virus des Schafes, MVV, und caprines Arthritis-Enzephalitis-Virus der Ziege, CAEV) sowie mit dem bovinen Immunschwäche-Virus des Rindes (BIV). Das FIV führt wie das HIV beim Menschen durch Abnahme der CD4-Lymphozyten im Blut im Verlauf der Erkrankung, sowie damit einhergehend zu einer zunehmenden Schwächung des Immunsystems (Torten et al., 1991). Wenn die Zahl der CD4-Lymphozyten im Blut eine gewisse Grenze unterschritten hat, kommt es zu einem völligen Versagen des Immunsystems, in dessen Folge die Katzen sterben oder eingeschläfert werden müssen. Die FIV-Infektion kommt weltweit vor, nicht nur bei Hauskatzen, sondern auch bei wilden Feliden wie zum Beispiel den Löwen in Ostafrika. Die Häufigkeit der FIV-Infektion bei der Hauskatze variiert allerdings von Land zu Land. In der Schweiz, Deutschland und Österreich sind zwischen 3 und 4 % der erkrankten Katzen, die dem Tierarzt zur Untersuchung vorgestellt werden, mit diesem Virus infiziert. In Frankreich und England sowie gewissen Gebieten der USA liegt die Rate bei kranken, infizierten Katzen zwischen 10 und 15 %, in Japan kann sie zum Beispiel bis zu 40 % ansteigen. Damit handelt es sich bei der FIV-Infektion um eine wichtige Krankheit von veterinärmedizinischer Bedeutung. Neben dem tierärztlichen Aspekt ist die FIV-Infektion aber auch für die Humanmedizin von Interesse, da sich diese Infektion aufgrund der großen Ähnlichkeit mit der HIV-Infektion des Menschen als Modell zum Studium der HIV-Infektion und möglicher Prävention und Therapiemöglichkeiten in hohem Masse eignet (Gardner, 1991; Jarrett et al., 1990). Für die Bekämpfung der FIV-Infektion sind Tierärzte und Tierbesitzer bislang ausschließlich auf den Nachweis der Infektion mittels immunologischer Tests und Abtrennung infizierter Tiere von nicht infizierten angewiesen. Eine Impfung, die es erlaubt, Katzen gegen die FIV Infektion zu schützen ohne gleichzeitig Antikörper zu induzieren, die zu falsch-positiven Reaktionen im Test führt, mit dem die FIV Infektion nachgewiesen wird, existiert zur Zeit nicht.

Der natürliche Schutz vor Infektionskrankheiten beruht auf der Wiedererkennung von Strukturen erfolgreich bekämpfter Erreger durch die Zellen des adaptiven Immunsystems. Dabei können zwei Hauptaktivitäten unterschieden werden. Auf der einen Seite ist dies die Aktivität des humoralen Immunsystems, welches auf der Synthese von Antikörpern durch B-Lymphozyten beruht. Neben dem humoralen Immunsystem kennt man auf der anderen Seite das zelluläre Immunsystem, welches auf der Aktivität vor allem von T-Lymphozyten beruht. Diese T-Lymphozyten sind in der Lage, mit Viren infizierte Körperzellen als "fremd" zu erkennen. Je nach spezieller Funktion der T-Zelle amplifiziert und modifiziert diese dann das Signal der Fremderkennung (T-Helferzellen), oder leitet direkt die Lyse der als fremd oder infiziert erkannten Zelle ein (zytotoxische T-Zellen). Für das Funktionieren der Immunantwort ist die korrekte Kooperation des humoralen mit dem zellulären lmmunsystem entscheidend. In den letzten zehn Jahren wurde klar, dass der zelluläre Arm des Immunsystems durch Aktivierung von sog. Typ-1-Helferzellen und der humorale Arm durch Aktivierung von sogenannten Typ-2- Helferzellen induziert wird (Mosmann et al., 1986). Entsprechend dieser Bezeichnung der Helferzellen wird der zelluläre Arm auch als TH1 pathway und der humorale Arm als TH2 pathway des Immunsystems bezeichnet.

Zur Lösung der einleitend geschilderten Probleme wurde auf die Immunisierung mit DNA zurückgegriffen (Ulmer JB et al. 1993). Diese mittlerweile intensiv erforschte Methodik bedient sich der Vakzinierung durch endogen exprimierte Antigene, welche intrazellulär verarbeitet und dem Immunsystem präsentiert werden. Neben bestimmten technischen bzw. ökonomischen Vorteilen, die die Immunisierung mit DNA gegenüber der Herstellung attenuierter oder rekombinanter Impfstoffe bietet, hat der Ansatz eine Reihe biologischer Vorteile. Vor allem fällt darunter die Möglichkeit, durch Ko-expression geeigneter Zytokine oder Beibringung signalvermittelnder Stoffe die Entstehung einer Typ-1 oder Typ-2-Antwort in den Immunzellen selbst zu beeinflussen.

Zur Beibringung der DNA-Expressionskonstrukte ist eine Reihe von Verfahren bekannt. Die der ballistischen Transfektion von Zielzellen ist in den Schriften WO91/00539, EP 0 500 799 beschrieben. Ein Apparat hierzu ist in WO95/19799 offenbart.

Die Impfung durch direkte Injektion nackter DNA ist in US 5,580,859, US 5,589,466 sowie US 5,593,972 offenbart.

Ein Nachteil der im Moment zur DNA-Immunisierung verwendeten Vektoren besteht darin, dass entweder Vektoren viralen Ursprungs eingesetzt werden, welche vom Aspekt der Sicherheit Probleme aufwerfen können (Gunzburg WH und Salmons B, 1995) oder Plasmide eingesetzt werden, die zwar epidemiologisch als vollkommen sicher zu gelten haben und immunologisch viralen Ansätzen weit überlegen sind, die allerdings dennoch Sequenzen enthalten, die zur Wirksamkeit der immunisierenden Expressionskassette keinen Beitrag liefern. Unter diese Sequenzen fallen alle zur Vermehrung in Bakterien benötigten Sequenzen wie Antibiotikaresistenzgene, Replikationsursprünge und ähnliche. Die Problematik wird in WO98/21322 ausführlich diskutiert.

Zudem ist die Entstehung von Immunantworten durch DNA-Immunisierung erheblich von der Menge und Beschaffenheit der inokulierten DNA abhängig. In Abhängigkeit von der Methode der Beibringung erfordert dabei die Immunisierung zwischen <1µg DNA und >100µg DNA bei Mäusen. Bei großen Mengen DNA kann die Anwesenheit von ISS (ISS = immunstimufatorischen Nukleinsäuresequenzen) wie den CpG-Sequenzen (CpG = unmethyliertes Cytosin-Guanosin) im Ampicillinresistenzgen die Qualität der Immunantwort erheblich beeinflussen, die ISS-Motive wurden überhaupt erst durch ein dies kontrollierendes Experiment zufällig gefunden (Krieg et al. (1995)). So wird bei DNA-Immunisierung mittels "Gene Gun", bei der sehr geringe Mengen DNA zur Immunisierung in den Körper des Patienten gebracht werden, typischerweise eher ein TH2-Profil der induzierten Immunantwort gefunden, während bei Injektion nackter DNA in den Muskel, bei der viel größere Mengen DNA eingesetzt werden, eher ein TH1-Profil beobachtet wird.

In bezug auf die immunstimulatorischen Nukleinsäuresequenzen ("ISS") ist seit einigen Jahren bekannt, dass bestimmte kurze Nukleinsäuresequenzen eine erhebliche physiologische Wirkung aufweisen können, indem sie über bisher unbekannte Mechanismen Effektorzellen des Immunsystems stark stimulieren. Diese ISS sind nur einige Basen lang und wirken nicht über die Expression von auf ihnen kodierten Proteinen. Die meisten bekannten immunmodifizierenden kurzen Oligodesoxyribonukleotidsequenzen ("ODN") enthalten ein unmethyliertes Cytosin-Guanosin-Motiv (CpG-Motiv). Es wird angenommen, dass die Erkennung dieses Motivs durch bisher nicht aufgeklärte Erkennungsmechanismen in der eukaryoten Zelle zu einer Art Notrufmechansimus führt, der eine gegen virale oder bakterielle Erreger gerichtete Reaktion auslöst (siehe ausführlich auch hinsichtlich weiterer Literaturzitate WO98/1810). Gemäß diesem Erklärungsvorschlag soll die Erkennung von CpG-Motiven, deren Vorkommen im Genom von Eukaryonten gegenüber dem von Prokaryonten erheblich unterdrückt ist, dem höheren Tier als "Wamsignal" dienen. Das Vorhandensein der CpG-Motive signalisiert nach dieser Theorie eine Infektion durch prokaryote Erreger, und deren Erkennung ermöglicht eine Bekämpfung unabhängig von bzw. vor der Ausbildung einer T-Hefferzefl-vermittelten Immunantwort. Die T-Helferzell-unabhängige Stimulierung und Proliferation von B-Zellen wird dadurch ermöglicht, dass für T-Zell- und B-Zell-Aktivierung notwendige kostimulatorische Signale, vor allem die Sekretion von Cytokinen der zellulären Typ 1-Antwort (TH1-spezifische Cytokine wie Interferon gamma, IL-2, IL-12), durch die CpG-abhängigen Signalwege bereitgestellt werden. Außerdem wird durch ISS die Aktivität von NK-Zellen und Makrophagen erhöht. Die Aktivität der Typ-2 Cytokine (IL-4, IL-10) wird demgegenüber unterdrückt, wahrscheinlich durch einen Antagonismus zwischen TH1- und TH2-Antwort (Lipford et al. 1997, Chu et al. 1997).

Es ist für HIV und andere Lentiviren postuliert worden, dass der Verlauf der Infektion wesentlich von der offenbar sehr früh nach Viruskontakt getroffenen "Entscheidung" des Immunsystems für eine Typ1- oder Typ2-Antwort abhängt (Romagnani et al., 1994). So findet man bei Patientengruppen, die nach Infektion relativ lange eine hohe CD4-Helferzellzahl im Blut zeigen, auch hohe zytotoxische Aktivität gegen HIV-spezifische Antigene. Es ist auch bei einigen, wahrscheinlich mit dem Virus in Kontakt gekommenen Patienten, die dennoch HIV-Antikörper-negativ blieben, ebenfalls eine HIV-spezifische Typ-1-Antwort gezeigt worden. Dies führte zu der Vermutung, dass die Induktion einer zytotoxischen Antwort gegen lentivirale Antigene im Wege einer Impfung die Infektion mit den entsprechenden Erregern verhindern könne. Ein Beweis dieser Vermutung - resp. ein tauglicher impfstoff - ist allerdings bisher nicht geliefert worden.

Aus dem Gebiet der Forschung zur humanen HIV-Infektion ist bekannt geworden, dass die Expression oder Blockade bestimmter Chemokinrezeptoren den Verlauf der Infektion der Zielzellen erheblich beeinflussen kann (Mackewicz CE, et al.1996). Es wurde weiterhin vermutet, dass auch die Expression von IL-16 zur Inhibition der HIV-Replikation beitrage und damit einen Beitrag zu einer möglichen Schutzwirkung liefern könne (Idziorek T, et al., 1998; Amiel et al. ,1999; Zhou et al., 1997)

Einen Impfstoff, mit dem Katzen unter Feldbedingungen gegen diese Infektion geschützt werden können und der mit dem Nachweisverfahren der Infektion nicht interferiert, gibt es - trotz zahlreicher Versuche - zur Zeit nicht. Es wurde versucht, Katzen gegen die FIV-Infektion zu impfen unter Verwendung von FIV-infizierten, in Kultur vermehrten Zellen, die vor Injektion mit Formalin inaktiviert wurden (Yamamoto et al., 1993; Yamamoto et al., 1991, siehe auch Yamamoto: US 5,275,813, 4.1.94, sowie US 5,510,106. 23.4.96). Eine ähnliche Erfindung ist auch aus Tompkins et al., US 5,413,927, bekannt. Solchermaßen geimpfte Katzen entwickelten jedoch Antikörper gegen die verschiedenen Virusproteine des FIV. Diese Antikörper reagieren mit den heute eingesetzten diagnostischen Verfahren (Tonelli, 1991), weshalb bei einer solchen Katze aufgrund eines Tests nicht unterschieden werden kann, ob die Katze an einer Infektion leidet oder vakziniert worden war. Um einen Impfstoff zur Verfügung zu haben, der bei den geimpften Tieren nicht zu einem falsch-positiven Test führt und damit keine vorbestehende Infektion mit dem FIV vortäuschen soll, wurde von verschiedenen Gruppen seit Jahren versucht, durch Verwendung eines mit Verfahren der Gentechnologie hergestellten Hüllglykoproteins des FIV und unter Einbezug geeigneter Adjuvantien einen Impfstoff zu entwickeln. Ein Erfinder der vorliegenden Anmeldung hatte dabei die ersten Experimente in dieser Richtung unternommen. Die ersten Versuche schützten zwar nicht vor einer Testinfektion, ließen aber erkennen, dass bei vakzinierten Tieren eine partielle Immunität vorgelegen hatte (Hofmann-Lehmann et al., 1995; Lutz et al., 1995). Ein späteres Experiment führte zu einem klar objektivierbaren Teitschutz bei einer der geimpften Gruppen (Leutenegger et al., 1998; Lutz et al., 1996). Ein weiteres Experiment, in welchem Katzen mit rekombinantem FIV-Hüllglykoprotein vakziniert wurden, welches vorgängig mit Lymphozyten zusammen mit Formalin inaktiviert worden war und unter Verwendung verschiedener Adjuvantien zur Immunisierung eingesetzt wurde, zeigte keinen Erfolg (Boretti and F., 1999). Die Arbeit anderer Forschergruppen über die Verwendung von rekombinantem Hüllglykoprotein als Vakzine-Antigen ist umfassend dokumentiert (Huisman et al., 1998; Osterhaus et al., 1996; Richardson et al., 1997). Allerdings gelang es auch diesen bislang nicht, unter Verwendung solcher Hüllglykoproteine oder der dafür kodierenden DNA einen Schutz zu erzielen (Osterhaus et al., 1996; Richardson et al., 1997; Richardson et al., 1998).

Rekombinante Impfstoffe gegen FIV sind weiterhin aus dem Patent von Wasmoen et al., US 5,849,303 bekannt.

Die Verwendung von IL-12 als Adjuvans bei Menschen ist aus US 5,571,515 bekannt.

Die Lehre von Gebrauch und Herstellung immunstimmulatorischer, CpG-enthaltender ISS ist in der Anmeldung WO 98/18810 sowie den darin zitierten Schriften umfassend erläutert.

Kovalent geschlossene hantelörmige Expressionskonstrukte aus DNA sind aus Wittig et al. (WO 98/21322) bekannt. Der Offenbarungsgehalt aller genannten Schriften soll als Teil der hier vorgelegten Beschreibung angesehen werden.

Trotz der vielen Arbeiten auf diesem Gebiet, gibt es bisher noch keinen guten und nachweisbaren Impfschutz für Katzen vor Infektion mit FIV.

Ziel und Aufgabe der vorliegenden Erfindung ist es deshalb, einen Impfstoff für Felide bereitzustellen, welcher einen Schutz gegen Erkrankung infolge von FIV-Infektion zu induzieren vermag, wobei die Impfung vorzugsweise so zu gestalten war, dass geimpfte Tiere durch ihren Antikörperstatus von erkrankten oder infizierten Tieren unterschieden werden können.

### Darstellung der Erfindung

Erfindungsgemäß wird dieses Ziel dadurch erreicht, dass Tiere, insbesondere Säugetiere wie Katzen, mit einer DNA-Sequenz immunisiert werden, welche das Hüllglykoprotein kodiert, sowie eines Hilfs-Polynukleotidsequenz, welche die für IL-16 codierende Sequenz unter der Kontrolle eines in entsprechenden Tier aktiven Promoters enthält. In einer bevorzugten Ausführungsform der Erfindung werden der Impfmischung geeignete Adjuvatien beigegeben, die eine zytotoxische Immunantwort hervorrufen, beispielsweise Zytokine der TH1-Antwort, Zytokinkodierende DNA-Expressionskonstrukte oder immunstimulatorische DNA Sequenzen.

Zur Lösung der einleitend geschilderten Probleme wurde auf die Immunisierung mit DNA zurückgegriffen, zu der bereits oben Ausführungen gemacht worden sind (siehe z.B. Ulmer JB et al. (1993), WO91/00539, EP 500799 ,WO95/19799, US 5,580,859, US 5,589,466 sowie US 5,593,972).

Die erfindungsgemäße Vakzine zur Schutzimpfung oder Therapie einer Lentiviren-Infektion, insbesondere der FIV-Infektion bei Katzen, zeichnet sich aus durch das Vorhandensein einer immunisierenden Polynukleotidsequenz, die mindestens einen Teil des Gens des Hüllproteins (env-Gen) des entsprechenden Lentivirus, insbesondere des FIV, unter der Kontrolle eines in dem entsprechenden Tier, insbesondere Katzen, operablen eukaryonten Promoters enthält oder daraus besteht kodiert, sowie eines Hilfs-Polynukleotidsequenz, welche die für IL-16 codierende Sequenz unter der Kontrolle eines in entsprechenden Tier aktiven Promoters enthält. Mindestens ein Teil des env-Gens bedeutet, dass ein oder mehrere Teile des env-Gens oder auch das ganze env-Gen vorhanden sind. Die Teile des env-Gens müssen zumindest einen so großen Teil des Hüllproteins codieren, dass eine Immunantwort hervorgerufen wird.

Eine solche Vakzine enthält vorzugsweise eine immunisierende Polynukleotidsequenz mit der die extrazellulär resp. außerhalb des Virus gelegene Domäne des env-Genprodukts sowie mindestens zwanzig Aminosäuren des Transmembrananteils des env-Genprodukts kodierenden Sequenz.

Nukleotidsequenzen, die in der vorliegenden Erfindung verwendbar sind und im Rahmen der Beispiele verwendet wurden, sind in SEQ ID NO 1 (FIV gp140), SEQ ID NO 2 (IL-12p40), SEQ ID NO 3 (IL-12p35), SEQ ID NO 4 (IL-16), SEQ ID NO 5 (CpG) und SEQ ID NO 6 (CpG) in einem gesonderten Sequenzprotokoll zu finden.

Eine bevorzugte Vakzine enthält als minimalen Teil der immunisierenden Polynukleotidsequenz, die unter SEQ ID NO 1 angegebene Sequenz oder eine mit der unter SEQ ID NO 1 angegebenen Sequenz zu mindestens 85% identische Sequenz oder eine Sequenz, die ohne die Entartung des genetischen Codes mit der in SEQ ID NO 1 angegebenen Sequenz zu mindestens 85 % identisch ist. Die Ähnlichkeit auf Nukleinsäurebasis wurde mit dem Programm MacMolly Complign Version 3.8 (www.mologen.com) und den Parametern "gap penalty = 3" sowie "mismatch penalty = 1" berechnet.

Selbstverständlich schließen die oben angegebenen Sequenzen auch die Komplementärsequenzen ein.

In einer Ausführungsform der Erfindung enthält die Vakzine zusätzlich mindestens eine Hilfs-Polynukleotidsequenzen. Diese Hilfs-Polynukleotidsequenz enthält die für IL-12 codierende Sequenz, insbesondere - für die Behandlung von Feliden, speziell Hauskatzen - die für beide Untereinheiten des felinen IL-12 codierende Sequenz, unter der Kontrolle eines oder mehrerer im entsprechenden Tier operablen eukaryonten Promoters.

In einer weiteren Ausführungsform der Vakzine kann diese die für IL-12 codierenden Sequenzen, z.B. die Sequenzen, die für die beiden Untereinheiten des felinen IL-12 codieren, und die Sequenz, die für II-16, z.B. das feline IL-16, codieren in der gleichen Hilfs-Polynukleotidsequenz und unter der Kontrolle eines oder mehrerer im entsprechenden Tier, z.B. der Katze, operablen eukaryonten Promoter enthalten.

In einer weiteren Ausführungsform enthält die Vakzine eine Hilfs-Polynukleotidsequenz, welche mindestens eine Sequenz der Basenfolge N¹N²CGN³N⁴ aufweist, wobei N¹N² ein Element aus der Gruppe GT, GG, GA, AT oder AA und N³N⁴ ein Element aus der Gruppe CT oder TT bedeutet. Dabei haben C, G, A und T die übliche Bedeutung, nämlich C Cytosin, G Guanin, A Adenin und T Thymin. Im anschließenden Beispiel wurde 5'-phosphoryliertes Oligodesoxynukleotid der Sequenz 5'-GTTCTTCGGG GCGTTCTTTT TTAAGAACGC CCC (SEQ ID NO 5) sowie ebenfalls 5'-phosphorytiertes Oligodesoxynukleotid der Sequenz 5'-GAAGAACGTT TTCCAATGAT TTTTCATTGG AAAAC (SEQ ID NO 6) in äquimolaren Mengen in einer Gesamtkonzentration von 1 mg DNA pro ml Lösung mit T4-DNA-Ligase in Anwesenheit von 1 mM ATP umgesetzt. Nach vollständiger Ligation wurden nichtumgesetzte Edukte durch Reaktion mit T7-DNA-Polymerase in Abwesenheit von dNTPs verdaut und das Produkt nach Reinigung über Anionen-Austausch-HPLC erhalten.

Bevorzugte Sequenzen, die in der Vakzine, insbesondere für die Behandlung von Katzen, enthalten sein können sind die Sequenzen gemäss SEQ ID NO 2 (IL-12), SEQ ID NO 3 (IL-12), SEQ ID NO 4 (IL-16),SEQ ID NO 5 (CpG).oder SEQ ID NO 6 (CpG).

Die Polynukleotidsequenzen resp. Expressionskonstrukte, sowohl der immunisierenden als auch der Hilfs-Polynukleotidsequenz können aus kovalent geschlossenen linearen Desoxyribonukleinsäuremolekülen bestehen, welche einen linearen Doppelstrangbereich aufweisen und welche doppelstrangbildenden Einzelstränge durch kurze einzelsträngige Schlaufen aus Desoxyribonukleinsäurenukleotiden verknüpft sind. Solche Konstrukte bestehen vorzugsweise nur aus der kodierenden Sequenz unter Kontrolle eines im Impfling operablen Promotors und einer Terminatorsequenz.

Ein weiterer Gegenstand der vorliegenden Erfindung ist ein Verfahren zur Impfung oder Therapie der Lentivireninfektion, insbesondere der FIV-Infektion bei Katzen, die dadurch gekennzeichnet ist, dass Polynukleotidsequenzen, wie sie oben beschrieben wurden, auf einem geeigneten Trägermaterial aufgebracht, auf die Haut des Tieres, insbesondere der Katze, beschleunigt wird, in die Zellen der Haut des Tieres eindringt und dort exprimiert wird. Ein bevorzugtes Trägermaterial ist Gold, wobei aber auch andere, massereiche und inerte Materialien geeignet sind.

Neben der oben beschriebenen Impfmethode für die Applikation der erfindungsgemäßen Impfstofizusammensetzungen können auch andere Applikationsmethoden eingesetzt werden, bei denen die in der Diskussion des Stands der Technik beschriebenen Nachteile zumindest zum Teil vermindert und vorzugsweise nicht vorhanden sind. Solche Applikationsmethoden sind beispielsweise Verabreichung der Polynukleotidsequenzen in wässriger Lösung z.B. durch Injektion in die Muskulatur.

Da die Rolle einer zytotoxischen Antwort theoretisch als wichtig angesehen wird und die Entstehung einer solchen Antwort von einer effizienten Genexpression mit möglichst geringer Beteiligung inadvertent exprimierter Proteine provoziert werden sollte, wurde im Rahmen des nachfolgenden Beispiels die Beibringung der Vakzine mittels ballistischem Transfer in Form von minimalistischen DNA-Expressionskonstrukten als gegenwärtig bevorzugte Beibringungsmethode verwendet.

Ziel des mittels des ballistischen Transfers durchgeführten Versuches war es, die Entwicklung der Immunantwort soweit wie möglich hinsichtlich der Komponenten zu differenzieren, die den essentiellen Beitrag zur Schutzwirkung liefern. Diese Aufgabe ist bei der Katze weitaus schwerer zu lösen als beispielsweise beim Menschen oder der Maus. Es kann bei immunologisch gut erforschten Spezies z.B. über die Typisierung der im Laufe der Impfung entstehenden Antikörper (IgG Subtyp 1 ist Immunantwort-Typ-2-spezifisch, Subtyp IgG2b ist Typ-1-spezifisch) viel über den zugrunde liegenden Antworttyp ausgesagt werden. Wenn man von der Tragfähigkeit des Typ-1/Typ-2-Paradigmas ausgeht, liefert diese Information Aufschlüsse hinsichtlich der Wirksamkeit und Richtung einer Vakzinierung, auch ohne dass ein Versuch der Infektion und Überprüfung des Schutzes stattgefunden hätte. Auf diese Weise lässt sich die bevorzugte Komposition und Beibringungsmethode zum Erreichen einer hypothetisch prophylaktischen Immunantwort mit viel geringerem Aufwand erreichen, als wenn jede Modalität der Impfung mit einem Infektions-/ Schutzexperiment untersucht wird. Es ist bei der Katze beim gegenwärtigen Wissensstand leider nicht möglich, solche Voraussagen mit dem gleichen Grad an Sicherheit zu treffen, wie dies bei Spezies wie dem Menschen oder der Maus der Fall wäre. Selbst grundlegende Paradigma wie die Typ1-Typ2 Dichtomie sind für die Katze nicht belegt. Die große Übereinstimmung vieler grundlegender Immuncharakteristika zwischen Mensch und Maus ist in vielen Fällen auf diese beiden Spezies beschränkt und nicht auf andere übertragbar. Um so überraschender war der große Erfolg der hier probierten Regimes.

Eine erfindungsgemäße Vakzine kann anstelle von Polynukleotiden auch Proteine resp. Glykoproteine enthalten, welche durch die in der vorliegenden Anmeldung, z.B. im Beispiel 1, beschriebenen Nukteotidsequenzen oder ähnliche Sequenzen kodiert werden. Ein auf Proteinbasis beruhendes Vakzineexperiment wurde in der Arbeit von Leutenegger et al (1998) im Detail beschrieben. In jenem Beispiel gelang es zwar, unter Verwendung eines handelsüblichen Adjuvans (QS-21) bei einigen Katzen einen Teilschutz zu erzielen, die Resultate waren aber unbefriedigend. Durch den Zusatz von Zytokinen (IL-12 und/oder IL-16) in der Form von Proteinen als Adjuvantien, gemäss der vorliegenden Erfindung, kann die Wirkung von Vakzinen auf Proteinbasis wesentlich verstärkt werden. Für solche Vakzinen geeignete Proteine, z. B. Proteine des FIV oder immunisierende Teile derselben, sowie die Zytokine IL-12 und IL-16 können in Bakterien (z.B. E.coli), in Hefen, in Säugerzellen (z.B.CHO-Zellen), in Insektenzellen (z.B. Zellen von Heliothis zea, ATCC No. CRL-9281) oder in Pflanzen (z.B. Tabakpflanze) exprimiert und daraus gereinigt werden.

Weitere vorteilhafte Maßnahmen sind in den übrigen Unteransprüchen enthalten. Die Erfindung wird nun anhand eines konkreten Beispiels, das die in den Ansprüchen definierte Erfindung aber in keiner Weise einschränken soll, sowie in den Figuren, weiter erläutert. Es zeigt.

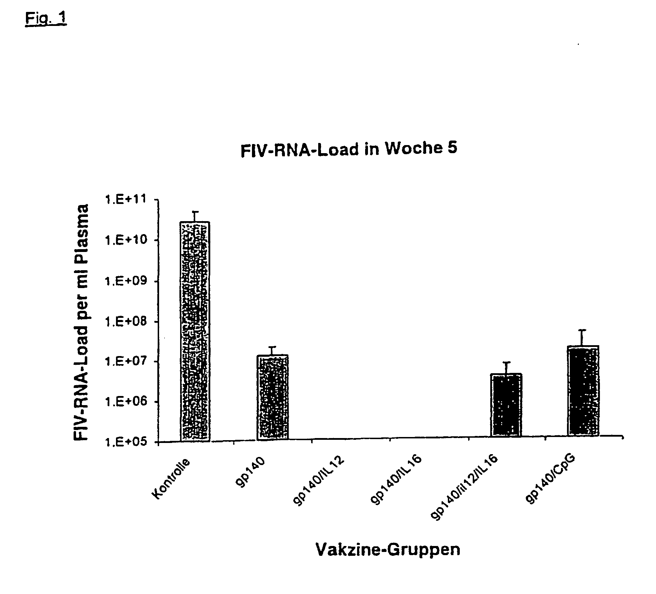

- **Figur 1**: zeigt die Resultate der FIV-RNA Quantifizierung im Plasma der Katzen.

### Ausführungsbeispiele

Es wurden fünf Impfregimes formuliert. Grundantigen in allen Gruppen (mit Ausnahme der Kontrollgruppe) war das gp140-SU-Antigen, das Hauptprodukt des env-Genes des FIV.

Das hier erwähnte Genkonstrukt soll generell als gp140-DNA bezeichnet werden. Um die Nützlichkeit dieses gp140-DNA-Konstruktes zu prüfen, wurde folgendes Experiment angestellt: Es wurden sechs Gruppen ä je vier Katzen eingesetzt, die mit verschiedenen Präparationen von gp140-DNA und ggf. DNA-kodierten Adjuvantien, in einem Fall auch nicht-kodierender adjuvanter DNA, immunisiert wurden. Die DNA-Konstrukte waren die oben erwähnten minimalistischen Expressionskonstrukte, bestehend lediglich aus der kodierenden Sequenz, vor welche noch die Sequenz des Zytomegalo-Virus-Promotors (CMV) gestellt worden war. Die kodierende Sequenz und der CMV-Promotor wurden als lineare Doppelstrangmoleküle verwendet, die beidseitig kovalent abgeschlossen wurden, um einer extra- oder intrazellulären Degradation durch Exonukteasen vorzubeugen. Die DNA-Konstrukte wurden auf kleine Goldpartikel adsorbiert, welche direkt in die Haut der Versuchstiere eingeschossen wurden. Die Tiere wurden dreimal im Abstand von drei Wochen mit den entsprechenden Konstrukten beschossen, wobei die DNA pro Schuss auf 1 mg Gold aufgezogen wurde. Zur Immunisierung wurde eine Helios gene-gun (Biorad, München, D) und ein Druck von 500 psi verwendet. Die gesamte DNA-Dosis betrug 2 µg pro Tier pro Impfung. Vier Wochen nach der dritten Immunisierung wurden die Tiere einer Testinfektion mit dem für die Gewinnung des Vakzineantigens verwendeten FIV-Stamm (Stamm Zürich 2, (Morikawa et al., 1991)) infiziert. Als Infektionsdosis für die Testinfektion wurde die 25fache Menge jener Konzentration verwendet, welche bei 50 % der Katzen zur Infektion führen würde (cat infective dose 50 = CID₅₀). Bei den verschiedenen Gruppen handelte es sich um die folgenden:

**Tabelle 1**

| Zusammensetzung der Vakzinegruppen | | |
|---|---|---|
| **Gruppe Nr.** | **Vakzine enthielt:** | **Fragestellung** |
| 1 | nur Goldpartikel | negative Kontrollen, bei diesen Katzen wurde kein Schutz erwartet |
| 2 | gp140-DNA | Wirksamkeit des gp140-DNA-Konstrukts allein |
| 3 | gp140-DNA + IL-12-DNA | Abklärung der Wirkung von IL-12 im Vergleich zur Gruppe 2 |
| 4 | gp140-DNA + IL-16-DNA | Abklärung der Wirkung von IL-16 im Vergleich zur gp140-DNA allein (Gruppe 2) |
| 5 | gp140-DNA + IL-12-DNA + IL-16-DNA | Abklärung des kombinierten Effektes der IL-12- und IL-16-DNA im Vergleich zur Gruppe 3 respektive Gruppe 4 |
| 6 | gp140-DNA + CpG | Abklärung des Effektes von CpG |

Der Schutzeffekt der verschiedenen Vakzine-Präparationen wurde durch Messung der folgenden Parameter jeweils in wöchentlichen Abständen bestimmt (Ausnahme: Messung des RNA Loads, nur in Woche 5):
1. Antikörper gegen das Transmembran-Protein (TM) wurden mittels eines ELISA-Verfahrens bestimmt.
2. Die Menge der FIV-RNA im Plasma dieser Katzen wurde mittels eines Taq-Man®-PCR-Verfahrens bestimmt.
3. Die Menge der in die DNA der Lymphozyten eingebauten FIV-DNA, die sogenannte Provirus-DNA, wurde mittels eines TaqMan®-Verfahrens bestimmt (Leutenegger et al. 1999).

Die Resultate können wie folgt zusammengefasst werden:
1. *Serokonversion gegen TM:* Der Verlauf der Serokonversion ist in Tabelle 2 zusammengefasst.
Daraus geht hervor, dass die Tiere der Kontrollgruppe 1 außerordentlich stark serokonvertierten, was auf eine hohe Virusreplikationsrate schließen lässt. Ab der fünften Woche waren hier vier von vier Tieren seropositiv.
In der Gruppe 2 kam es nur allmählich zur Serokonversion, und der Grad der Serokonversion war wesentlich geringer als in der Gruppe 1. In der Gruppe 2 waren auch noch in der neunten Woche nicht alle Tiere seropositiv. Dies lässt auf eine verringerte Virusvermehrung schließen, was mit einem Schutz vereinbar ist.
In der Gruppe 3 serokonvertierte bis in die siebte Woche lediglich ein Tier, die anderen blieben völlig negativ. Dies deutet darauf hin, dass bei drei von vier Tieren ein kompletter Schutz erzielt worden ist. Beim einen positiven Tier kam es aufgrund der im Vergleich zu den Tieren der Gruppe 1 verringerten Serokonversion nur zu einer mäßigen Virusvermehrung.
In der Gruppe 4 serokonvertierten bis zur siebten Woche zwei von vier Tieren. Auch hier kam es nur zur Bildung geringer Antikörpermengen, was ebenfalls auf eine geringgradige Virusvermehrung schließen lässt. Die Kombination von IL-12 und IL-16 erwies sich dagegen - was die Serokonversionsrate anbelangt - als weniger wirksam. Hier zeigten bis zur siebten Woche vier von vier Tieren Serokonversion.
Für die Gruppe 6 gilt die gleiche Aussage wie für die Gruppe 5. Auch hier serokonvertierten vier von vier Tieren bis zur siebten Woche.

**Tabelle 2**

| FIV Vakzine Experiment, TM-ELISA Resultate | | | | | | | | | | | | | | |
|---|---|---|---|---|---|---|---|---|---|---|---|---|---|---|
| | | **Wochen nach Testinfektion** | | | | | | | | | | | | |
| **Katze** | **Gruppe** | **-7** | **-5** | **-3** | **0** | **1** | **2** | **3** | **4** | **5** | **6** | **7** | **8** | **10** |
| 2916 | 1 | 0,7 | 0 | 0 | 0 | 0,3 | 0,1 | 0 | 1,6 | 70,9 | 92,2 | 86,2 | 85,9 | 85,9 |
| 2932 | | 1,2 | 0,3 | 0,2 | 0 | 0,8 | 0,4 | 0 | 1,6 | 59,8 | 78,6 | 96,2 | 65,6 | 65,6 |
| 381 | | 0,6 | 1,3 | 0,1 | 0 | 0,4 | 0,2 | 0 | 5,3 | 78 | 95,3 | 72,6 | 89,3 | 89,3 |
| 384 | | 0 | 1 | 0,1 | 0 | 0,4 | 0,7 | 0 | 10,7 | 83,8 | 90,7 | 78,7 | 79,4 | 79,4 |
| 2924 | 2 | 0 | 0,3 | 0 | 0 | 0,04 | 0,8 | 0,4 | 1,9 | 76,4 | 94,3 | 88,7 | 88,3 | 88,3 |
| 2947 | | 0 | 1 | 0,5 | 0 | 0,4 | 0,6 | 0 | 0 | 0,4 | 1,3 | 0 | 60,4 | 60,4 |
| 379 | | 0,6 | 0,9 | 0,1 | 0 | 0 | 0,4 | 0 | 0,4 | 0,8 | 16,7 | 31,6 | 82,4 | 82,4 |
| 393 | | 0 | 0,3 | 0 | 0 | 0 | 0 | 0 | 0,8 | 66,7 | 85,7 | 63,6 | 90 | 90 |
| 2917 | 3 | 0 | 1,2 | 0,2 | 0 | 0 | 0,2 | 0 | 0 | 0,1 | 1,1 | 0 | 0,9 | 0,9 |
| 2943 | | 0 | 0,8 | 0 | 0 | 0 | 0,8 | 0 | 0 | 0 | 0,9 | 0 | 0,8 | 0,8 |
| 377 | | 1 | 2,4 | 2.3 | 0 | 2,6 | 0,4 | 0 | 1,6 | 0 | 2,1 | 0 | 0,5 | 0,5 |
| 388 | | 0 | 0,2 | 0 | 0 | 0,4 | 0,1 | 0 | 0 | 54,6 | 86,6 | 68,8 | 80,1 | 80,1 |
| 2922 | 4 | 0 | 0,5 | 0 | 0 | 0,5 | 0 | 0 | 0 | 0 | 2,6 | 0 | 1,1 | 1,1 |
| 2933 | | 0 | 0,7 | 0 | 0 | 0,3 | 0,1 | 0 | 0 | 6,7 | 88,2 | 86,6 | 80,4 | 80,4 |
| 382 | | 0,7 | 1,4 | 1,4 | 0 | 0 | 0,7 | 0 | 1 | 0,1 | 1,4 | 3 | 64,3 | 64,3 |
| 394 | | 0,2 | 1 | 0,4 | 0 | 0 | 0,3 | 0 | 0,4 | 0,05 | 39,9 | 45,7 | 74,6 | 74,6 |
| 2923 | 5 | 0 | 0,2 | 0 | 0 | 0 | 0,2 | 0 | 0,9 | 2,6 | 49,4 | 41,3 | 74,4 | 74,4 |
| 2959 | | 0,08 | 0,6 | 0 | 0 | 0 | 0,3 | 0 | 47,5 | 88,5 | 97,2 | 93,4 | 79,1 | 79,1 |
| 378 | | 0,4 | 1,9 | 0,2 | 3,6 | 0 | 0,3 | 0 | 2,9 | 50 | 99,2 | 77,4 | 81,6 | 81,6 |
| 389 | | 0,08 | 0,2 | 0 | 0 | 0 | 0,2 | 0 | 0,3 | 38,3 | 89,4 | 73,3 | 97,8 | 97,8 |
| 2925 | 6 | 0,08 | 0,6 | 0 | 0,8 | 0 | 0,1 | 0 | 5 | 63,1 | 80 | 68 | 79,4 | 79,4 |
| 2936 | | 0,3 | 1,2 | 0,2 | 0 | 0 | 0,4 | 0 | 0 | 8,1 | 61,2 | 64,2 | 60,2 | 60,2 |
| 380 | | 1,4 | 7,4 | 0,1 | 0 | 0 | 0,4 | 0 | 0 | 1,3 | 70,3 | 56,8 | 71,8 | 71,8 |
| 385 | | 1,6 | 9,5 | 1,2 | 0 | 0,7 | 0,2 | 0 | 1,3 | 9,7 | 108,3 | 65,4 | 87,2 | 87,2 |

2. *FIV-RNA-Virusload im Plasma:* Die Resultate der FIV-RNA-Quantifizierung im Plasma der Katzen sind in Figur 1 zusammengestellt. Die Resultate können wie folgt kommentiert werden:
Gruppe 1: Hier war der RNA-Virusload am höchsten.
Gruppe 2: Die mit der gp140-DNA vakzinierten Katzen zeigten einen signifikant geringeren RNA-Virusload als die Tiere der Kontrollen; daraus kann abgeleitet werden, dass allein der gp140-Konstrukt schon einen Teilschutz zu induzieren vermag. Diese Resultate sind in Übereinstimmung mit der Serologie.
Gruppe 3: Zufügung von IL-12 zur gp140-DNA zeigte bis zur fünften Woche einen vollständigen Schutz der Katzen gegen Virus im Blut. Auch diese Resultate stimmen mit den Beobachtungen der Serologie überein.
Gruppe 4: Die Zugabe von IL-16-DNA führte ebenfalls zu einem eindrücklichen Schutz, indem bis zur fünften Woche auch hier keine Viren im Plasma nachgewiesen werden konnten.
Gruppe 5: Die Zugabe von IL-12 und IL-16 erwies sich als weniger gut als IL-12 oder IL-16 allein. In der fünften Woche konnten bei drei von vier Tieren RNA im Plasma nachgewiesen werden. Allerdings war auch hier die Menge gegenüber den Kontrollkatzen der Gruppe 1 signifikant geringer.
Gruppe 6: Die Verwendung der CpG zeigte ebenfalls einen Teilschutz auf. Allerdings ließen sich in der fünften Woche bei allen Tieren geringe Mengen von FIV-RNA nachweisen.

3. *Menge der proviralen DNA:* Die Resultate der Quantifizierung der proviralen DNA-Menge aller Katzen ist in Tabelle 3 zusammengestellt. Die Resultate lassen sich wie folgt kommentieren:
Gruppe 1: Wie bei der Serologie und der RNA-Messung erwiesen sich auch hier die Tiere der Gruppe 1 als voll empfänglich für die Testinfektion.
Gruppe 2: Auch die Tiere der Gruppe 2 wurden ausnahmslos Provirus-positiv, die mittlere FIV-Provirus-Menge war jedoch nur unwesentlich geringer als jene der Kontrollgruppe.
Gruppe 3: Wie zuvor für die Serologie und die RNA-Menge beobachtet, erwies sich auch hier die Menge der proviralen DNA als am geringsten. Nur 1 von 4 Tieren wurde Provirus-positiv, die andern 3 wurden überhaupt nicht infiziert.
Gruppe 4: Auch die IL-16-DNA führte zu einem verminderten Provirus-Load, der auch in der sechsten Woche noch signifikant geringer war als jener der Kontrolle.
Gruppe 5: Erstaunlicherweise erwies sich der Provirus-Load bei Tieren, die die Kombination von IL-12 und IL-16 erhalten hatten, als sehr gering. Daraus kann abgeleitet werden, dass IL-12 und IL-16 auf die Integration der Provirus-Menge einen signifikanten Schutzeffekt aufweist.
Gruppe 6: Erstaunlicherweise war der Effekt der CpG-Zugabe auf die Provirus-Menge ebenfalls außerordentlich ausgeprägt. In der sechsten Woche ließen sich nur Spuren von integriertem Provirus nachweisen. Diese Resultate stehen etwas im Widerspruch zu den Befunden der RNA-Messung. Sie lassen sich eventuell dadurch erklären, dass CpGs mithelfen, eine Integration des Virus in die DNA der Wirtszelle zu hemmen.

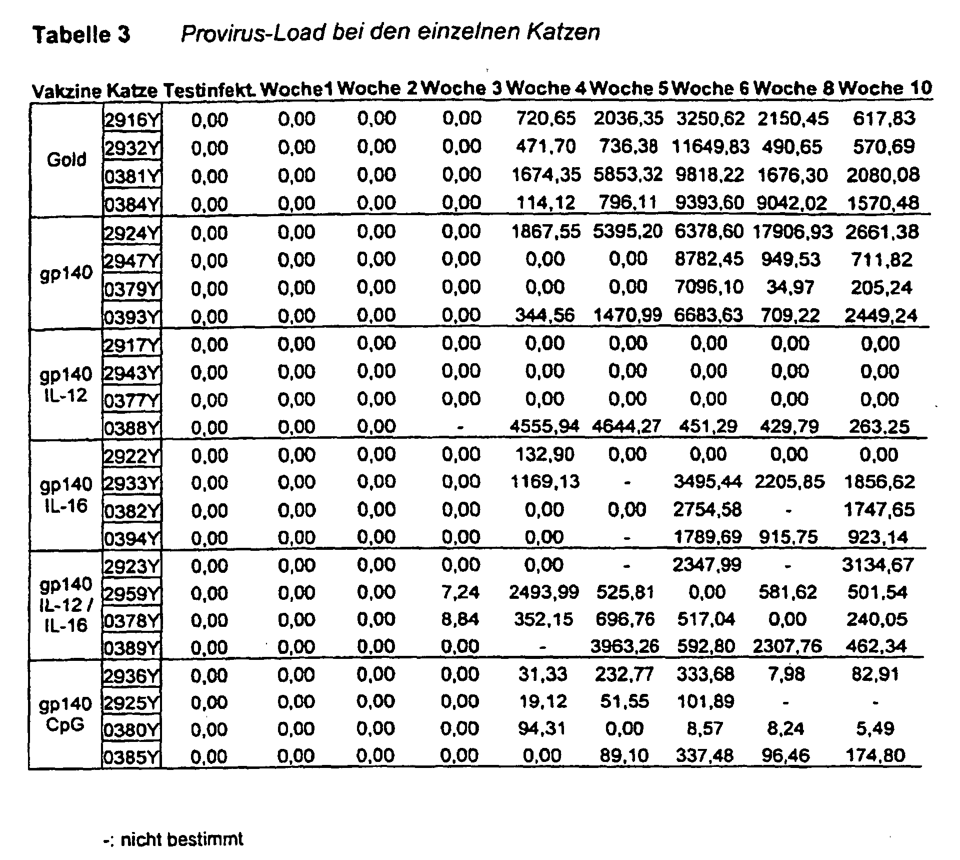

Zusammenfassend kann festgehalten werden, dass überraschenderweise festgestellt wurde, dass die Verwendung von gp140-DNA und verschiedener Zusätze einen unterschiedlich hohen Schutzeffekt zu induzieren vermag. Der Schutzeffekt äußert sich einerseits in einer geringeren Virusreplikation, was zu geringem oder vollständigem Fehlen der Serokonversion führt und/oder zu geringerer Integration der viralen DNA in die Wirtszell-DNA.

### Literaturverzeichnis

Amiel C, Darcissac E, Truong MJ, Dewulf J, Loyens M, Mouton Y, Capron A, Bahr GM; (1999) Interleukin-16 (IL-16) inhibits human immunodeficiency virus replication in cells from infected subjects, and serum IL-16 levels drop with disease progression. J Infect Dis, 179(1):83-91

Boretti and F. (1999) Experimentelle Immunisierung von Katzen mit rekombinanten Hüllgykoproteinen des Felinen Immunschwächevirus: Einfluss verschiedener ImpfstoffForlulierungen auf die Immunantwort und Vergleich mit einer Ganzvirusvakzine. Vet.-Diss. Zürich,

Chu, R.S., Targoni, O.S., Krieg, A.M., Lehmann, P.V. and Harding, C.V. (1997) CpG oligodeoxynucleotides act as adjuvants that switch on T helper 1 (Th1) immunity. Journal of Experimental Medicine 186(10), 1623-31

Gardner, M.B. (1991) Simian and feline immunodeficiency viruses: animal lentivirus models for evaluation of AIDS vaccines and antiviral agents. [Review] [82 refs]. Antiviral Research 15(4), 267-86.

Gunzburg WH, Salmons B, (1995) Virus vector design in gene therapy.Mol Med Today 1(9):410-7)

Hofmann-Lehmann, R., Holznagel, E., Aubert, A., Bauer-Pham, K. and Lutz, H. (1995) FIV vaccine studies. II. Clinical findings, hematological changes and kinetics of blood lymphocyte subsets. Veterinary Immunology & Immunopathology 46(1-2), 115-25.

Huisman, W., Karlas, J.A., Siebelink, K.H., Huisman, R.C., de Ronde, A., Francis, M.J., Rimmelzwaan, G.F. and Osterhaus, A.D. (1998) Feline immunodeficiency virus subunit vaccines that induce virus neutralising antibodies but no protection against challenge infection. Vaccine 16(2-3), 181-7.

Idziorek T, Khalife J, Billaut-Mulot O, Hermann E, Aumercier M, Mouton Y, Capron A, Bahr GM (1998) Recombinant human IL-16 inhibits HIV-1 replication and protects against activation-induced cell death (AICD) Clin Exp Immunol;112(1):84-91

Jarrett, O., Yamamoto, J.K. and Neil, J.C. (1990) Feline immunodeficiency virus as a model for AIDS vaccination. [Review] [15 refs]. Aids 4(Suppl 1), S163-5.

Krieg AM, Yi AK, Matson S, Waldschmidt TJ, Bishop GA, Teasdale R, Koretzky GA, Klinman DM (1995) CpG motifs in bacterial DNA trigger direct B-cell activation, Nature 6;374(6522):546-9.

Leutenegger, C.M., Hofmann-Lehmann, R., Holznagel, E., Cuisinier. A.M., Wolfensberger, C., Duquesne, V., Cronier, J., Allenspach, K., Aubert, A., Ossent, P. and Lutz, H. (1998) Partial protection by vaccination with recombinant feline immunodeficiency virus surface glycoproteins. AIDS Research & Human Retroviruses 14(3), 275-83.

Lutz, H., Hofmann-Lehmann, R., Bauer-Pham, K., Holznagel, E., Tozzini, F., Bendinelli, M., Reubel, G., Aubert, A., Davis, D., Cox, D. and et al. (1995) FIV vaccine studies. I. Immune response to recombinant FIV env gene products and outcome after challenge infection. Veterinary Immunology & Immunopathology 46(1-2), 103-13.

Lutz, H., Hofmann-Lehmann, R., Leutenegger, C., Allenspach, K., Cuisinier, A.M., Cronier, J., Duquesne, V. and Aubert, A. (1996) Vaccination of cats with recombinant envelope glycoprotein of feline immunodeficiency virus: decreased viral load after challenge infection. [Review] [17 refs]. AIDS Research & Human Retroviruses 12(5), 431-3.

Lipford, G.B., Bauer, M., Blank, C., Reiter, R., Wagner, H. and Heeg, K. (1997) CpG-containing synthetic oligonucleatides promote B and cytotoxic T cell responses to protein antigen: a new class of vaccine adjuvants. European Journal of Immunology 27(9), 2340-4.

Mackewicz CE, Barker E, Levy JA, (1996) Role of beta-chemokines in suppressing HIV replication. Science, 22;274(5291):1393-5

Morikawa, S., Lutz, H., Aubert, A. and Bishop, D.H. (1991) Identification of conserved and variable regions in the envelope glycoprotein sequences of two feline immunodeficiency viruses isolated in Zurich, Switzeriand. Virus Research 21(1), 53-63.

Osterhaus, A.D., Tijhaar, E., Huisman, R.C., Huisman, W., Darby, I.H., Francis, M.J., Rimmetzwaan, G.F. and Siebelink, K.H. (1996) Accelerated viremia in cats vaccinated with recombinant vaccinia virus expressing envelope glycoprotein of feline immunodeficiency virus. AIDS Research & Human Retroviruses 12(5), 437-41.

Pedersen, N.C., Ho, E.W., Brown, M.L. and Yamamoto, J.K. (1987) Isolation of a T-lymphotropic virus from domestic cats with an immunodeficiency-like syndrome. Science 235(4790), 790-3.

Richardson, J., Moraillon, A., Baud, S., Cuisinier, A.M., Sonigo, P. and Pancino, G. (1997) Enhancement of feline immunodeficiency virus (FIV) infection after DNA vaccination with the FIV envelope. Journal of Virology 71(12), 9640-9.

Richardson, J., Moraillon, A., Crespeau, F., Baud, S., Sonigo, P. and Pancino, G. (1998) Delayed infection after immunization with a peptide from the transmembrane glycoprotein of the feline immunodeficiency virus. Journal of Virology 72(3), 2406-15.

Romagnani, S., Maggi, E. and Del Prete, G. (1994) An alternative view of the Th1/Th2 switch hypothesis in HIV infection. AIDS Research & Human Retroviruses 10(5), iii-ix.

Tonelli, Q.J. (1991) Enzyme-linked immunosorbent assay methods for detection of feline leukemia virus and feline immunodeficiency virus. [Review] [7 refs]. Journal of the American Veterinary Medical Association 199(10), 1336-9.

Torten, M., Franchini, M., Barlough, J.E., George, J.W., Mozes, E., Lutz, H. and Pedersen, N.C. (1991) Progressive immune dysfunction in cats experimentally infected with feline immunodeficiency virus. Journal of Virology 65(5), 2225-30.

Ulmer JB, Donnelly JJ, Parker SE, Rhodes GH, Felgner PL, Dwarki VJ, Gromkowski SH, Deck RR, De Witt CM, Friedman A et al. (1993) Heterologous protection against influenza by injection of DNA encoding a viral protein. Science 259: 1745-1749).

Yamamoto, J.K., Hohdatsu, T., Olmsted, RA., Pu, R., Louie, H., Zochlinski, H.A., Acevedo, V., Johnson, H.M., Soulds, G.A. and Gardner, M.B. (1993) Experimental vaccine protection against homologous and heterologous strains of feline immunodeficiency virus. Journal of Virology 67(1), 601-5.

Yamamoto, J.K., Okuda, T., Ackley, C.D., Louie, H., Pembroke, E., Zochlinski, H., Munn, R.J. and Gardner, M.B. (1991) Experimental vaccine protection against feline immunodeficiency virus. AIDS Research & Human Retroviruses 7(11), 911-22.

Zhou P, Goldstein S, Devadas K, Tewari D, Notkins AL, (1997) Human CD4+ cells transfected with IL-16 cDNA are resistant to HIV-1 infection: inhibition of mRNA expression. Nat Med 3(6):659-64.

## Patentansprüche

1. Vakzine zur Schutzimpfung oder Therapie einer Lentiviren-Infektion bei Feliden, **dadurch gekennzeichnet, dass** sie eine immunisierende Polynukleotidsequenz enthält, die mindestens ein Teil des Gens des Hüllproteins (env-Gen), des entsprechenden Lentivirus, unter der Kontrolle eines in dem entsprechenden Tier operablen eukaryoten Promoters enthält oder daraus besteht, und dass sie eine Hilfs-Polynukleotidsequenz enthält, welche die für IL-16 codierende Sequenz unter der Kontrolle eines im entsprechenden Tier operablen eukaryoten Promoters enthält.

2. Vakzine nach Anspruch 1, **dadurch gekennzeichnet, dass** sie eine Hilfs-Polynukleotidsequenz enthält, welche die für IL-12 und IL-16 codierenden Sequenzen unter der Kontrolle eines oder mehrerer im entsprechenden Tier operablen eukaryoten Promoter enthält.

3. Vakzine nach Anspruch 2, **dadurch gekennzeichnet, dass** die Hilfs-Polynukleotidsequenz eine Sequenz enthält, die für beide Untereinheiten des felinen IL-12 und für das feline IL-16 codiert und dass diese Sequenzen unter der Kontrolle eines in Katzen operablen eukaryoten Promoters sind.

4. Vakzine nach einem der Ansprüche 1 bis 3, **dadurch gekennzeichnet, dass** sie eine Hilfs-Polynukleotidsequenz enthält, welche mindestens eine Sequenz der Basenfolge N¹N²CGN³N⁴ aufweist, wobei N¹N² ein Element aus der Gruppe GT, GG, GA, AT oder AA und N³N⁴ ein Element aus der Gruppe CT oder TT ist.

5. Vakzine nach wenigstens einem der Ansprüche 1 bis 4, **dadurch gekennzeichnet, dass** die immunisierenden Polynukleotidsequenzen und die Hilfs-Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen und welche doppelstrangbildenden Einzelstränge durch kurze einzelsträngige Schlaufen aus Desoxyribonukleinsäurenukleotiden verknüpft sind, und welche Doppelstrangbildenden Einzelstränge nur aus der kodierenden Sequenz unter Kontrolle eines im zu impfenden Tier operablen Promotors und einer Terminatorsequenz besteht.

6. Vakzine gemäss einem der Ansprüche 1 bis 5, **dadurch gekennzeichnet, dass** die Hilfs-Polynukleotidsequenz eine kodierende Sequenz gemäss SEQ ID NO 8 (IL-12 p40), eine kodierende Sequenz gemäss SEQ ID NO 9 (IL-12 p35), eine kodierende Sequenz SEQ ID NO 10 (IL-16), SEQ ID NO 5 (CpG) oder SEQ ID NO 6 (CpG) oder eine zu einer dieser Sequenzen komplementäre Sequenz enthält.

7. Vakzine gemäss einem der Ansprüche 1 bis 6 zur Impfung oder Therapie einer Lentivirusinfektion bei Tieren, **dadurch gekennzeichnet, dass** eine immunisierende Polynukleotidsequenz und eine Hilfs-Polynukleotidsequenz gemäss einem der Ansprüche 1 bis 4 auf einem geeigneten massereichen und inerten Trägermaterial aufgebracht vorliegt, derart, dass sie auf die Haut des Tieres beschleunigt, in die Zellen der Haut des Tieres eindringen und dort exprimiert werden kann.

8. Vakzine gemäss Anspruch 7, **dadurch gekennzeichnet, dass** das Tier zur Gattung der Feliden gehört und vorzugsweise eine Hauskatze ist.

9. Vakzine gemäss Anspruch 7 oder 8, **dadurch gekennzeichnet, dass** das Trägermaterial Gold ist.

10. Vakzine zur Schutzimpfung oder Therapie einer Lentivirus-Infektion bei Feliden, **dadurch gekennzeichnet, dass** sie ein immunisierendes Hüllprotein oder einen immunisierenden Teil dieses Proteins, des entsprechenden Lentivirus zusammen mit IL-16 je als Protein enthält.

11. Vakzine gemäss Anspruch 10, **dadurch gekennzeichnet, dass** IL-12 als Protein enthält.

## Claims

1. Vaccine for vaccinating or treating a lentiviral infection in felids, **characterized in that** it comprises an immunizing polynucleotide sequence which contains, or consists of, at least a part of the gene for the envelope protein (env gene) of the corresponding lentivirus under the control of a eukaryotic promoter which is operable in the corresponding animal, and **in that** it comprises an auxiliary polynucleotide sequence which contains the IL-16-encoding sequence under the control of a eukaryotic promoter which is operable in the corresponding animal.

2. Vaccine according to Claim 1, **characterized in that** it comprises an auxiliary polynucleotide sequence which contains the IL-12- and IL-16-encoding sequences under the control of one or more eukaryotic promoter(s) which is/are operable in the corresponding animal.

3. Vaccine according to Claim 2, **characterized in that** the auxiliary polynucleotide sequence contains a sequence which encodes both subunits of the feline IL-12, and encodes the feline IL-16, and **in that** these sequences are under the control of a eukaryotic promoter which is operable in cats.

4. Vaccine according to one of Claims 1 to 3, **characterized in that** it comprises an auxiliary polynucleotide sequence which exhibits at least one sequence having the base sequence N¹N²CGN³N⁴, with N¹N² being an element from the group GT, GG, GA, AT or AA and N³N⁴ being an element from the group CT or TT.

5. Vaccine according to at least one of Claims 1 to 4, **characterized in that** the immunizing polynucleotide sequences and the auxiliary polynucleotide sequences are present as expression constructs which consist of covalently closed linear deoxyribonucleic acid molecules which exhibit a linear double-stranded region, and which double strand-forming single strands are linked by short single-stranded loops composed of deoxyribonucleic acid nucleotides, and which double strand-forming single strands only consist of the coding sequence under the control of a promoter which is operable in the animal to be vaccinated and a terminator sequence.

6. Vaccine according to one of Claims 1 to 5, **characterized in that** the auxiliary polynucleotide sequence contains a coding sequence as depicted in SEQ ID NO 8 (IL-12 p40), a coding sequence as depicted in SEQ ID NO 9 (IL-12 p35), a coding sequence as depicted in SEQ ID NO 10 (IL-16), SEQ ID NO 5 (CpG) or SEQ ID NO 6 (CpG) or a sequence which is complementary to one of these sequences.

7. Vaccine according to one of Claims 1 to 6 for vaccinating or treating a lentiviral infection in animals, **characterized in that** an immunizing polynucleotide sequence and an auxiliary polynucleotide sequence according to one of Claims 1 to 4 have been applied to a suitable mass-rich and inert support material such that, on the skin of the animal, they can penetrate into the cells of the skin of the animal in an accelerated manner and be expressed in these cells.

8. Vaccine according to Claim 7, **characterized in that** the animal belongs to the felid genus and is preferably a domestic cat.

9. Vaccine according to Claim 7 or 8, **characterized in that** the support material is gold.

10. Vaccine for vaccinating or treating a lentiviral infection in felids, **characterized in that** it comprises an immunizing envelope protein, or an immunizing part of this protein, of the corresponding lentivirus together with IL-16, in each case as protein.

11. Vaccine according to Claim 10, **characterized in that** it comprises IL-12 as protein.

## Revendications

1. Vaccin pour l'immunisation préventive ou le traitement d'une infection à lentivirus chez des félidés, **caractérisé en ce qu'**il contient une séquence polynucléotidique immunisante qui contient au moins ou consiste en au moins une partie du gène de la protéine d'enveloppe (gène env), du lentivirus correspondant, sous le contrôle d'un promoteur d'eucaryote fonctionnel chez l'animal correspondant, et **en ce qu'**il contient une séquence polynucléotidique auxiliaire qui contient la séquence codant pour IL-16 sous le contrôle d'un promoteur d'eucaryote fonctionnel chez l'animal correspondant.

2. Vaccin selon la revendication 1, **caractérisé en ce qu'**il contient une séquence polynucléotidique auxiliaire qui contient les séquences codant pour IL-12 et IL-16 sous le contrôle d'un ou plusieurs promoteur(s) d'eucaryote fonctionnel(s) chez l'animal correspondant.

3. Vaccin selon la revendication 2, **caractérisé en ce que** la séquence polynucléotidique auxiliaire contient une séquence qui code pour les deux sous-unités de l'IL-12 féline et pour l'IL-16 féline et **en ce que** ces séquences sont sous le contrôle d'un promoteur d'eucaryote fonctionnel chez les chats.

4. Vaccin selon l'une quelconque des revendications 1 à 3, **caractérisé en ce qu'**il contient une séquence polynucléotidique auxiliaire qui comporte au moins une séquence présentant la succession de bases N¹N²CGN³N⁴, N¹N² étant un élément choisi dans le groupe constitué par GT, GG, GA, AT et AA et N³N⁴ étant un élément choisi dans le groupe constitué par CT et TT.

5. Vaccin selon au moins l'une des revendications 1 à 4, **caractérisé en ce que** les séquences polynucléotidiques immunisantes et les séquences polynucléotidiques auxiliaires se trouvent sous forme de produits d'expression qui consistent en molécules d'acide désoxyribonucléique linéaires fermées par covalence qui comportent une région double brin linéaire et dont les simples brins formant le double brin sont reliés par des boucles simple brin à base de nucléotides d'acide désoxyribonucléique, et dont les simples brins formant le double brin ne sont constitués que de la séquence codante sous le contrôle, d'un promoteur fonctionnel chez l'animal à vacciner et d'une séquence de terminaison.

6. Vaccin selon l'une quelconque des revendications 1 à 5, **caractérisé en ce que** la séquence polynucléotidique auxiliaire contient une séquence codante selon SEQ ID n° 8 (p40 d'IL-12), une séquence codante selon SEQ ID n° 9 (p35 d'IL-12), une séquence codante SEQ ID n° 10 (IL-16), SEQ ID n° 5 (CpG) ou SEQ ID n° 6 (CpG) ou une séquence complémentaire de l'une de ces séquences.

7. Vaccin selon l'une quelconque des revendications 1 à 6 pour l'immunisation ou le traitement d'une infection à lentivirus chez des animaux, **caractérisé en ce qu'**une séquence polynucléotidique immunisante et une séquence polynucléotidique auxiliaire selon l'une quelconque des revendications 1 à 4 se trouvent appliquées sur une matière porteuse massive et inerte, de sorte que sur la peau de l'animal elles peuvent pénétrer plus rapidement dans les cellules de la peau de l'animal et y être exprimées.

8. Vaccin selon la revendication 7, **caractérisé en ce que** l'animal appartient à la famille des félidés et de préférence est un chat domestique.

9. Vaccin selon la revendication 7 ou 8, **caractérisé en ce que** la matière porteuse est de l'or.

10. Vaccin pour l'immunisation préventive ou le traitement d'une infection à lentivirus chez des félidés, **caractérisé en ce qu'**il contient en tant que protéine une protéine d'enveloppe immunisante, ou une partie immunisante de cette protéine, du lentivirus correspondant, conjointement avec IL-16.

11. Vaccin selon la revendication 10, **caractérisé en ce qu'**il contient en tant que protéine IL-12.
